# EUROPEAN PATENT SPECIFICATION

(11) **EP 1 254 634 B1**
(45) Date of publication and mention of the grant of the patent: **23.07.2003**
(21) Application number: 01850081.9
(22) Date of filing: 03.05.2001
(51) Int. Cl.: A61B 17/00

(54) **Guiding tool for wound closure element**
Führungswerkzeug für Wundverschluss
Outil de guidage pour fermeture de plaie

(43) Date of publication of application: 06.11.2002
(73) Proprietor: RADI MEDICAL SYSTEMS AB, 754 50 Uppsala (SE)
(72) Inventor: Akerfeldt, Dan, 755 92 Uppsala (SE); Egnelöv, Per, 754 40 Uppsala (SE); Preinitz, Fredrik, 753 50 Uppsala (SE)
(74) Representative: Lindgren, Anders

(56) References cited:
- WO-A-01/13800
- US-A- 5 620 461
- US-A- 5 649 950
- US-A- 5 725 519

## Description

The present invention relates generally to sealing of a percutaneous incision or puncture in the wall of a vessel, duct, lumen or hollow organ in the body of a living being, by positioning a sealing device in said incision or puncture, where the incision or puncture is smaller than said sealing device. In particular the invention relates to a guiding tool for enabling the correct positioning of such a sealing device, ensuring a leak-proof sealing.

### Background of the Invention

In recent years a number of devices and apparatuses have been developed enabling the closure or occlusion of e.g. punctures in the femoral artery following catheterization. Instead of applying a pressure to the puncture site for a period of time sufficient for blood clot formation to occur, the new methods are e.g. based on providing a plug, commonly referred to as an "artery plug", in the puncture. The plug is made of a resorbable material, such that it can be left in place until the tissue has recovered properly and the wound or puncture is healed. The plug can be made of collagen, and applied to the outside of a vessel against a counteracting element, also made of a resorbable material, introduced into the interior of the vessel. A locking means secures the collagen plug in place. A device of this kind is disclosed in US-5,935,147 (Kensey et al). The sealing action is thus performed by the externally applied collagen plug. However, a certain percentage of applied plugs will not be leak-proof and often further compression by other devices or manually must be applied.

Alternatively, a plug can be made of two members such that a first member is positioned within a vessel and acts as the occluding member, and a second member is positioned outside the vessel and locked to the first member by a locking means. In order to ensure leak-proof action, the first member is larger than the puncture in all directions, i.e. it will cover a surface larger than the area of the puncture. In order to make this possible, the first member is foldable. A device of this kind is disclosed in our own EP-application EP-00850184.3 (corresponding to US patent application serial No. 09/704,726).

US-A-5 649 950 discloses a loading device for a collapsible occluder. This device has a conical lumen. US-A-5 649 950 corresponds to the preamble of claim 1.

The problem facing all systems wherein a folding or deformation of an element is needed in order to introduce the element into a vessel or through any tissue wherein the hole is smaller than the element itself, is that it can be difficult to achieve a reproducible unfolding, that accurately seals the hole from the inside. Also, in many cases the closure element is provided inside an introducer member such as a tube, in a folded state, already at the time of manufacture of the kit comprising all components. If the kit is stored for extended periods of time, and even for shorter times, the folded closure element most likely does not unfold properly at the time of use. To avoid the risk of permanent deformation, the closure element could be inserted into the introducer device by the physician, but this would require an extra manipulation, and it might be very difficult to maintain the sterility of the devices in such a case.

### Summary of the Invention

Thus, in view of the problem with the prior art devices, it is the object of the present invention to improve the rate of successful sealing operations using wound closure devices, when foldable or deformable closure elements are introduced into a vessel through a hole, smaller than the element itself, and to enable the folding and unfolding operation to take place during the sealing operation, and to avoid manipulation of the closure element by the physician at the time of performing the sealing operation.

This object is achieved by the provision of a guiding tool which in a controlled manner deforms or folds a wound closure element such that it after having been introduced through a puncture, unfolds and regains its original shape in a reproducible and controllable manner, and thereby provides adequate sealing at an excellent rate of success, this procedure taking place at the time of performing the sealing operation. The guiding tool according to the invention is defined in claim 1.

### Brief Description of the Drawings

Fig. 1 a-c is a schematic illustration of a system for wound closure during operation thereof;
Fig. 2a illustrates an embodiment of the guiding tool not belonging to the invention in cross section;
Fig. 2b is a view in longitudinal cross section of a second embodiment of the inventive guiding tool;
Fig. 2c illustrates cross sections at A-E in Fig. 2b;
Fig. 2d is a perspective cut view in two different directions of a third preferred embodiment of the inventive guiding tool;
Fig. 3a is a cross section view of a closure element; and
Fig. 3b is a top view of a closure element.

### Detailed Description of the Invention

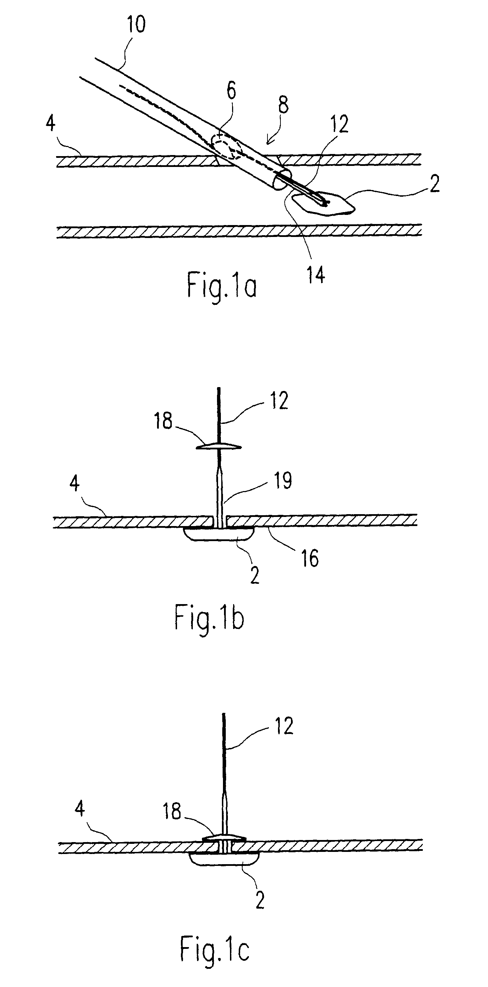

Fig. 1a-c illustrates the procedure of inserting a wound closure (occlusion) element 2 in a blood vessel 4 and clamping it by means of a locking member 6. Thereby, an incision 8 is made in the blood vessel 4 in question, and an introducer tube 10 is inserted into the vessel 4, see Fig. 1a. Then, a folded, or in some other manner deformed closure element 2 is passed through said introducer tube 10 and into the vessel 4. The closure element 2 is secured to e.g. a suture 12, and some rigid elongated element 14 such as a steel pin or pusher rod (not shown) can be used to guide the closure element 2 through the tube 10 and into the vessel 4, where it unfolds. Once the closure element 2 has unfolded inside the vessel 4, the pusher rod and the introducer tube 10 are withdrawn from the vessel but maintained close to the exterior of the vessel 4. Then, the closure element 2 is pulled back using the suture 12, so as to be located in a position where it is held against the interior vessel wall 16, Fig. 1b. An essentially disk shaped locking element 18, having a central hole, is provided on the suture 12, the suture running through said hole, such that the locking member can be moved along the suture 12 to be brought into contact with the exterior vessel wall 20. By the provision of suitable friction enhancing means 19 on the very distal end of the suture, the locking element can be pushed against the wall of the vessel while engaging the portion of the suture having higher friction, and thereby cause a locking of the closure element, Fig. 1c. This principle is used in e.g. US-5,916,236.

It is important that the closure element unfolds in a reproducible way, such that it will contact the inner vessel wall around the circumference of the incision. If the closure element is delivered to the user in a folded state, in position inside the introducer, as a "kit" ready for use by a physician, and the kit has been stored on a shelf for some time, it may happen that the closure element has become permanently deformed, and will not assume the desired shape (which may be to regain its original shape) inside the vessel. Such an event would of course dramatically increase the risk that the closure element will not fulfill its function, and cause a leakage.

Therefore, in accordance with the present invention, there is provided a guiding tool that is connectable to an introducer of the type discussed above, the closure element being provided in said guiding tool in an unfolded state. Thereby the actual deformation or folding of the closure element, necessary to enable insertion through the incision (which is smaller than the closure element) and into the blood vessel, is not performed until the time of performing the operation of inserting the closure element. In this way, the closure element will not be subjected to a prolonged deformation during storage.

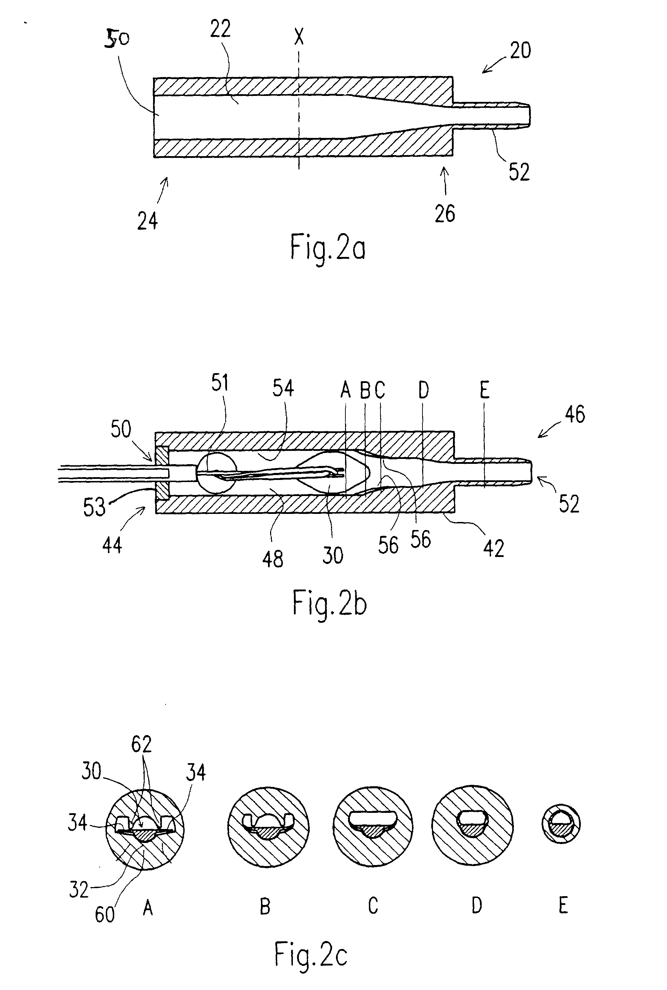

An embodiment not falling under the invention is illustrated in Fig. 2a. It comprises a body in the shown embodiment in the form of an elongated tube like member 20 (although cylindrical outer symmetry is no requirement), having an inner lumen 22 that has a first diameter in the proximal end portion 24 and over a fraction of its length (up to the dotted line X), forming a space 26 in which an closure element (not shown) can be housed without being deformed. Over a second fraction of the length, from the dotted line X and up to the distal end 26 portion, the lumen becomes narrower, rendering the 22 lumen cone shaped over this portion. Finally there is a connection portion 28, connectable to an introducer tube (such as the tube 10 in Fig. 1a). The exit diameter is equal to or slightly smaller than the inner diameter of the introducerbe, i.e. small enough to enable passing the closure element into the blood vessel.

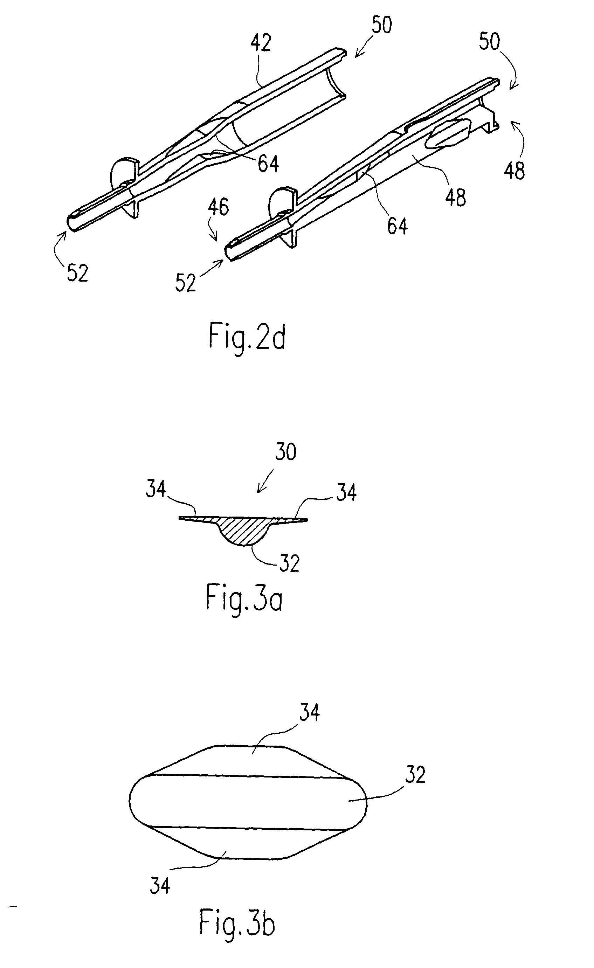

In Fig. 2b a second embodiment of the guiding tool according to the present invention is illustrated. It is adapted for a closure element 30 having the general shape shown in Figs. 3a and 3b. The closure element suitable for use with the illustrated embodiment of the guiding tool, has a thick mid portion 32, that is generally elongated, and has peripheral wings 34 or edges, which are substantially thinner, and thus more flexible than the mid portion 32. The peripheral shape is like a slightly distorted ellipse, but could in principle be circular, the wings 34 thereby forming rather a collar or a brim surrounding the mid portion. Other shapes are also possible. A suitable kind of closure element is disclosed and claimed in our copending EP-00850184.3. In view of its flexibility, the wings 34 or brim are foldable such that the entire element will have a smallest dimension in a folded state that fits well within an incision or puncture in a blood vessel, thereby enabling insertion into said vessel.

Thus, the embodiment of the guiding tool according to the present invention shown in Fig. 2b, comprises an essentially tubular element 42 having a proximal end 44 and a distal end 46 and a lumen 48 extending between said distal 46 end and said proximal end 44. There is further a wound closure element introduction opening 50 in the proximal end 44, and a wound closure element exit opening 52 in the distal end 46. The distal end is shaped so as to be connectable to an introducer tube, like the one described above in connection with Fig. 1.

The lumen 48 has inner walls 54 provided with wound closure element deformation surfaces 56, adapted to deform a wound closure element during passage thereof, past said surfaces 56, through the guiding tool, from an essentially undeformed or unfolded state to a deformed or folded state. The effect of this deformation/folding should be such that the overall dimension of the wound closure element is changed to render it capable of passing through the puncture in the blood vessel. Also, after passage through said incision or puncture it must regain a shape that is capable of providing a sealing action against the inner wall of the vessel.

In Fig. 2b there is also shown a pusher rod 51, having a fork like configuration, that is used to push the closure element 30 through the introducer and into the blood vessel. This pusher rod is retracted once the element is properly located inside the vessel.

In the embodiment shown in Fig. 2b the deformation surfaces 56 have a specific design, illustrated by a sequence in Fig. 2c, which are cross section views through the lumen at the positions indicated with corresponding letters A, B, C, D, E in Fig. 2b. Thus, there is one guiding surface 56 for each wing on the closure element 30, and a guiding recess 60 in which the thicker mid portion 32 of the closure element 30 will run during its passage through the guiding tool. The guiding surfaces will initially have an orientation such that the wings 34 of the unfolded or undeformed closure element 30, when inserted into the guiding tool in the introduction opening, will rest thereon in a position and orientation causing no deformation (referred to as a "horizontal" orientation; position A in Fig. 2c). Thereby the nominal shape of the closure element 30 is preserved also during extended storage. In a direction towards the exit opening, the guiding surfaces will gradually become elevated (A-B-C-D-E in Fig 2c) from the initial, essentially horizontal orientation, and also curved such that they form what could be referred to as a "quasi-conical" lumen inside the tool. At the end of the lumen, near the exit opening, the guiding surfaces will have reached a state where the cross section of the lumen is essentially circular, and where the diameter corresponds to the inner diameter of the introducer tube.

Preferably, there are guiding rails 62 provided above the guiding surfaces 56, such that the wings or brims 34 of the closure element 30 will be kept down during the process of pushing it with the pusher rod through the guiding tool, thereby preventing inadvertent tilting or incorrect behavior of the closure element during the movement through said guiding tool. The rails are preferably integrated in the "roof" of the lumen. However, they can also be provided as an insert and attached by suitable means inside the lumen.

In a further variation of the above described embodiment, the interior lumen of the guiding tool essentially has a cross section that exactly corresponds to the cross section of the closure element. This lumen would then form a guiding slot inside the tool, whereby the slot would be shaped so as to gradually change from a cross section corresponding to the above mentioned "horizontal orientation" to an essentially circular cross section, the diameter of which would be smaller than the incision through which the closure element is to be inserted. There must however be a space above the closure element for the access by the pushing rod needed for advancing the closure element through the tool and the introducer.

The sequence A-E in Fig. 2c illustrates the folding process, and it is clearly seen that the closure element reaches a folded state where it conforms to the circular cross section of the introducer tube, which is connected to the guiding tool at the exit end thereof.

Another embodiment of the guiding tool is shown in Fig. 2d, showing perspective cuts through the device at two orientations perpendicular to each other. Like reference numerals are used for like elements in Figs. 2c and 2d. Like in the above described embodiment, this embodiment of the guiding tool comprises an essentially tubular element 42 having a proximal end 44 and a distal end 46 and a lumen 48 extending between said distal 46 end and said proximal end 44. There is further a wound closure element introduction opening 50 in the proximal end 44, and a wound closure element exit opening 52 in the distal end 46. The distal end is shaped so as to be connectable to an introducer tube.

Also in this embodiment, the lumen 48 has inner walls 54 also provided with wound closure element deformation surfaces 56, adapted to deform a wound closure element during passage thereof, past said surfaces 56, through the guiding tool, from an essentially undeformed state to a deformed state.

However, the deformation surfaces have a different design in this embodiment. Namely, one wall of the inner lumen is flat such that the closure element can be placed essentially flat thereon, in an unfolded state, e.g. during shelf storage, corresponding to the "horizontal orientation" discussed above in connection with Fig. 2b, but with the thicker mid portion facing upwards. Towards the exit opening the lumen is shaped as cone, much like in the first embodiment of Fig. 2a, whereby the final diameter of the inner lumen corresponds to a diameter smaller than the diameter of the opening in the blood vessel through which the closure element is to be introduced. About half-way along the conical portion of the lumen, there are provided a pair of deflection surfaces 64, having a "steeper" angle than the over-all cone angle of the lumen. These surfaces will engage the peripheral brim or collar portions of the closure element, such that they are deflected or bent downwards from the essentially flat initial position. Also, the pusher rod discussed above will cause the closure element not to lie completely flat, but slightly angled with the trailing edge at a slightly elevated position compared to the leading edge. Furthermore, said pusher rod will assist in keeping the leading edge forced against the flat surface.

In order to facilitate correct positioning of the closure element during insertion, also in this case there may be provided guiding rails like those described above.

The invention having been described with reference to preferred embodiments thereof can be subject to alterations and modifications by the man skilled in the art, and the scope of the invention is limited only by the appended claims.

## Claims

1. A guiding tool for controllable and reversible folding or deformation of a wound closure element (2) before insertion of said wound closure element into a percutaneous incision or puncture in the wall (4) of a vessel, duct, lumen or hollow organ in the body of a living being, said incision or puncture being smaller than said wound closure element in an unfolded or undeformed state, said tool comprising a body (20) having a distal end (26) and a proximal end (24) and a lumen (22) extending between said distal end and said proximal end; a wound closure element introduction opening (50) in the proximal end, and a wound closure element exit opening (52) in the distal end;
**characterized in that**
said lumen has inner walls (54) provided with wound closure element guiding surfaces (56, 64) in the form of separate deflection elements protruding from the inner wall of said lumen, for reversibly reducing the spatial extension of a wound closure element during its passage through the guiding tool, by engaging a peripheral brim or collar portions of the closure element, such that they are deflected or bent downwards from an essentially flat initial position, whereby said wound closure element is capable of passing through said incision or puncture, and whereby it after passage through said incision or puncture assumes a shape that is capable of providing a sealing action against said incision or puncture.

2. The tool as claimed in claim 1, wherein said guiding surfaces (56) in the proximal end of said tool are essentially flat, and in a direction towards the distal end, gradually becomes elevated and also curved such that they form a quasi-conical lumen inside the tool, and wherein the guiding surfaces, at the distal end of the lumen, near the exit opening, will have reached a state where the cross section of the lumen is essentially circular.

3. The tool as claimed in claim 1 or 2, wherein there are guiding rails (62) provided above the guiding surfaces (56), such that wings or rims (34) of a closure element (30) will be kept down during the process of pushing through the guiding tool, said guiding rails (62) being arranged to prevent the closure element from inadvertent tilting or other incorrect behavior during its movement through the guiding tool.

4. The tool as claimed in claim 1, wherein said introduction opening is larger than said exit opening, and optionally is provided with a sealing plug (53) to prevent leakage of body fluid.

5. The tool as claimed in claim 1, wherein said guiding surfaces (56) gradually change from the proximal end, where they do not affect the nominal shape of the wound closure element (30), towards the distal end where they force the wound closure element (30) to assume a shape that conforms to the inner lumen of an introducer tube, connectable to the distal end of said guiding tool.

6. The tool as claimed in claim 1, wherein said lumen is cone shaped at least in the distal region of said guiding tool, and has a large enough diameter in the proximal region of the guiding tool, that the nominal shape of the wound closure element (30) is not affected, and a small enough diameter in the distal end that the wound closure element conforms to the inner lumen of an introducer tube, connectable to the distal end of said guiding tool.

7. The tool as claimed in claim 1, wherein the proximal region of the tool is essentially cylindrical, forming a storage compartment for said closure element (30), in which the nominal shape of the wound closure element is not affected.

8. A system for the introduction and securing of a wound closure element into a percutaneous incision or puncture in the wall of a vessel, duct, lumen or hollow organ in the body of a living being, said incision or puncture being smaller than said wound closure element in an unfolded or undeformed state, said system comprising
a wound closure element (30);
an introducer tube (10) for introducing said wound closure element into said incision;
a pusher device for enabling the passage of said wound closure element through said introducer; and
a tool as claimed in claim 1, connectable to said introducer.

## Patentansprüche

1. Führungswerkzeug zum gesteuerten und reversiblen Falten oder Verformen eines Wundverschlusselements (2) vor der Einführung dieses Wundverschlusselements in einen perkutanen Schnitt oder eine perkutane Punktur in der Wand (4) eines Gefäßes, eines Kanals, eines Lumens oder eines hohlen Organs im Körper eines Lebewesens, wobei der Schnitt oder die Punktur kleiner ist als das Wundverschlusselement in einem ungefalteten oder nichtverformten Zustand, wobei das Werkzeug einen Körper (20) mit einem distalen Ende (26) und einem proximalen Ende (24) sowie einem Lumen (22) aufweist, das sich zwischen dem distalen und dem proximalen Ende erstreckt, eine Einführöffnung (50) für das Wundverschlusselement in dem proximalen Ende und eine Ausgangsöffnung (52) für das Wundverschlusselement in dem distalen Ende;
**dadurch gekennzeichnet, dass**
das Lumen Innenwände (54) hat, die mit Führungsflächen (56, 64) für das Wundverschlusselement in Form von separaten Ablenkelementen versehen sind, die von der Innenwand des Lumens hervorstehen, um die räumliche Ausdehnung eines Wundverschlusselements während seines Durchgangs durch das Führungswerkzeug durch Ineingriffbringen eines Außenrands oder Kragenbereichs des Verschlusselements reversibel zu reduzieren, so dass diese aus einer im Wesentlichen flachen anfänglichen Position abgelenkt oder nach unten gebogen werden, wobei das Wundverschlusselement durch den Schnitt oder die Punktur hindurchtreten kann und wobei es nach dem Durchtritt durch den Schnitt oder die Punktur eine Gestalt annimmt, die eine Dichtungswirkung gegen den Schnitt oder die Punktur bieten kann.

2. Werkzeug nach Anspruch 1, wobei die Führungsflächen (56) am proximalen Ende des Werkzeugs im Wesentlichen flach sind und in Richtung des distalen Endes nach und nach erhaben und auch gekrümmt werden, so dass sie ein quasi konisches Lumen innerhalb des Werkzeugs bilden, und wobei die Führungsflächen am distalen Ende des Lumens in der Nähe der Ausgangsöffnung einen Zustand erreicht haben werden, wo der Querschnitt des Lumens im Wesentlichen kreisförmig ist.

3. Werkzeug nach Anspruch 1 oder 2, wobei Führungsschienen (62) oberhalb der Führungsflächen (56) vorgesehen sind, so dass Flügel oder Kanten (34) eines Verschlusselements (30) während des Vorgangs des Hindurchdrückens durch das Führungswerkzeug unten gehalten werden, wobei die Führungsschienen (62) so angeordnet sind, dass ein unbeabsichtigtes Kippen oder anderweitiges unkorrektes Verhalten des Verschlusselements während seiner Bewegung durch das Führungswerkzeug hindurch verhindert wird.

4. Werkzeug nach Anspruch 1, wobei die Einführöffnung größer ist als die Ausgangsöffnung und optional mit einem Dichtungsverschluss (53) versehen ist, um ein Auslaufen von Körperflüssigkeit zu verhindern.

5. Werkzeug nach Anspruch 1, wobei die Führungsflächen (56) sich von dem proximalen Ende, wo sie die Nenngestalt des Wundverschlusselements (30) nicht beeinflussen, in Richtung des distalen Endes, wo sie das Wundverschlusselement (30) dazu zwingen, eine Gestalt einzunehmen, die sich dem inneren Lumen einer Einführröhre anpasst, verbindbar mit dem distalen Ende des Führungswerkzeugs, nach und nach verändern.

6. Werkzeug nach Anspruch 1, wobei das Lumen zumindest im distalen Bereich des Führungswerkzeugs kegelförmig ist und im proximalen Bereich des Führungswerkzeugs einen Durchmesser hat, der groß genug ist, um die Nenngestalt des Wundverschlusselements (30) nicht zu beeinflussen, und im distalen Ende einen Durchmesser hat, der klein genug ist, damit sich das Wundverschlusselement an das innere Lumen einer Einführröhre, verbindbar mit dem distalen Ende des Führungswerkzeugs, anpasst.

7. Werkzeug nach Anspruch 1, wobei der proximale Bereich des Werkzeugs im Wesentlichen zylindrisch ist und ein Aufbewahrungsabteil für das Verschlusselement (30) bildet, wobei die Nenngestalt des Wundverschlusselements nicht beeinträchtigt wird.

8. System zum Einführen und Sichern eines Wundverschlusselements in einen perkutanen Schnitt oder eine perkutane Punktur in der Wand eines Gefäßes, eines Kanals, eines Lumens oder eines hohlen Organs im Körper eines Lebewesens, wobei der Schnitt oder die Punktur kleiner ist als das Wundverschlusselement in einem ungefalteten oder nicht-deformierten Zustand, wobei das System Folgendes aufweist:
ein Wundverschlusselement (30) ;
eine Einführröhre (10) zum Einführen des Wundverschlusselements in den Schnitt;
eine Druckeinrichtung zum Ermöglichen des Durchgangs des Wundverschlusselements durch die Einführröhre hindurch; und
ein Werkzeug nach Anspruch 1, das mit der Einführröhre verbindbar ist.

## Revendications

1. Outil de guidage pour le repliement ou la déformation contrôlables et réversibles d'un élément de fermeture de plaie (2) avant l'insertion dudit élément de fermeture de plaie dans une incision percutanée ou un orifice dans la paroi (4) d'un vaisseau, d'un canal, d'une ouverture ou d'un organe creux dans le corps d'un être vivant, où ladite incision ou ledit orifice est plus petit que ledit élément de fermeture de plaie à l'état non replié ou non déformé, ledit outil comprenant un corps (20) avec une extrémité distale (26) et une extrémité proximale (24) et une ouverture (22) entre lesdites extrémités distale et proximale, une ouverture d'introduction (50) de l'élément de fermeture de plaie à l'extrémité proximale et une ouverture de sortie (52) de l'élément de fermeture de plaie à l'extrémité distale, **caractérisé en ce que** ledit passage présente des parois internes (54) offrant des surfaces de guidage (56, 64) de l'élément de fermeture de plaie sous la forme d'éléments déflecteurs distincts disposés en saillie sur la paroi interne dudit passage et destinés à réduire de manière réversible l'extension dans l'espace d'un élément de fermeture de plaie pendant son passage dans l'outil de guidage en agissant sur un bord ou collet périphérique de l'élément de fermeture de manière à ce qu'il soit fléchi où replié vers le bas à partir d'une position initiale essentiellement plane, si bien que ledit élément de fermeture de plaie peut passer au travers de ladite incision ou dudit orifice et, après son passage dans ladite incision ou ledit orifice, reprendre une forme telle qu'il soit en mesure d'exercer une action de fermeture sur ladite incision ou ledit orifice.

2. Outil selon la revendication 1, où lesdites surfaces de guidage (56) dans l'extrémité proximale dudit outil sont essentiellement planes et, en direction de l'extrémité distale, remontent et se courbent progressivement de manière à former une ouverture pratiquement conique à l'intérieur de l'outil et où les surfaces de guidage, à l'extrémité distale de l'ouverture, près de l'ouverture de sortie, atteignent une position telle que la section de l'ouverture est essentiellement circulaire.

3. Outil selon la revendication 1 ou 2, où il existe au-dessus des surfaces de guidage (56) des rails de guidage (62) tels que les ailes ou bords (34) de l'élément de fermeture (30) sont maintenus en position basse pendant l'opération de poussée au travers de l'outil de guidage, lesdits rails de guidage (62) étant disposés de manière à éviter que l'élément de fermeture ne puisse inopinément basculer ou mal se positionner pendant son mouvement au travers de l'outil de guidage.

4. Outil selon la revendication 1, où ladite ouverture d'introduction est plus grande que ladite ouverture de sortie et peut en option être dotée d'un bouchon étanche (53) destiné à éviter l'épanchement des fluides corporels.

5. Outil selon la revendication 1, où lesdites surfaces de guidage (56) changent progressivement, entre l'extrémité proximale, où elles n'affectent pas la forme nominale de l'élément de fermeture de plaie (30), et l'extrémité distale, où elles forcent l'élément de fermeture de plaie (30) à prendre une forme correspondant à l'ouverture interne d'un tube d'introduction pouvant être connecté à l'extrémité distale dudit outil de guidage.

6. Outil selon la revendication 1, où ledit passage est de forme conique, au moins dans la région distale dudit outil de guidage, et possède un diamètre suffisamment grand dans la région proximale de l'outil de guidage pour que la forme nominale de l'élément de fermeture de plaie (30) ne soit pas affectée et un diamètre suffisamment petit dans la région distale pour que l'élément de fermeture de plaie corresponde à l'ouverture interne d'un tube d'introduction pouvant être connecté à l'extrémité distale dudit outil de guidage.

7. Outil selon la revendication 1, où la région proximale de l'outil est essentiellement cylindrique, formant un compartiment de stockage pour ledit élément de fermeture de plaie (30), où la forme nominale de celui-ci n'est pas affectée.

8. Système pour l'introduction et la fixation d'un élément de fermeture de plaie dans une incision percutanée ou un orifice dans la paroi d'un vaisseau, d'un canal, d'une ouverture ou d'un organe creux du corps d'un être vivant, ladite incision ou ledit orifice étant plus petit que ledit élément de fermeture de plaie à l'état non replié ou non déformé, ledit système comprenant
un élément de fermeture de plaie (30),
un tube d'introduction (10) pour introduire ledit élément de fermeture de plaie dans ladite incision,
un dispositif de poussage permettant de faire passer ledit élément de fermeture de plaie dans ledit tube d'introduction, et
un outil selon la revendication 1, pouvant être connecté audit tube d'introduction.
